# EUROPEAN PATENT SPECIFICATION

(11) **EP 2 180 902 B1**
(45) Date of publication and mention of the grant of the patent: **23.11.2011**
(21) Application number: 08787477.2
(22) Date of filing: 26.08.2008
(51) Int. Cl.: A61K 49/10, A61K 49/20, G01N 33/50

(54) **IMAGING MEDIUM COMPRISING HYPERPOLARISED ¹³C-ACETATE AND USE THEREOF**
BILDGEBUNGSMEDIUM MIT HYPERPOLARISIERTEM ¹³C-ACETAT UND SEINE VERWENDUNG
MILIEU D'IMAGERIE COMPRENANT DE L'ACETATE-¹³C HYPERPOLARISE ET SON UTILISATION

(30) Priority: 27.08.2007 NO 20074367
(43) Date of publication of application: 05.05.2010
(73) Proprietor: GE Healthcare Limited, Little Chalfont Buckinghamshire HP7 9NA (GB)
(72) Inventor: GISSELSSON, Anna, SE-227 63 Lund (SE); HANSSON, Georg, SE-235 35 Vellinge (SE); MÅNSSON, Sven, SE-237 34 Bjärred (SE); IN'T ZANDT, René, SE-247 36 Södra Sandby (SE); KARLSSON, Magnus, SE-212 43 Malmö (SE); JENSEN, Pernille, R., DK-2200 København N (DK); LERCHE, Mathilde, H., DK-1903 Fredriksberg C (DK)
(74) Representative: Wulff, Marianne Weiby
(86) International application number: PCT/EP2008/061129
(87) International publication number: WO 2009/027388

(56) References cited:
- WO-A-92/01937
- WO-A-99/35508
- WO-A-2007/064226
- WO-A-2007/069909
- WO-A-2007/111515
- WO-A-2007/134160
- WO-A-2008/026937
- WO-A-2008/086534
- WO-A1-2008/086534
- SZCZEPANIAK LIDIA ET AL: "Oxidation of acetate in rabbit skeletal muscle: Detection by 13C NMR spectroscopy in vivo" MAGNETIC RESONANCE IN MEDICINE, vol. 36, no. 3, 1996, pages 451-457, XP002514948 ISSN: 0740-3194
- R.R. RAMSAY, V.A. ZAMMIT: "Carnitine acyltransferases and their influence on CoA pools in health and disease" MOLECULAR ASPECTS OF MEDICINE, vol. 25, 2004, pages 475-493, XP002514949
- P.R. JENSEN ET AL.: "Acetyl-CoA and acetyl-carnitine show organ specific distribution in mice after injection of DNP hyperpolarised 13C1 acetate" PROC. INTL. MAG. RESON. MED., vol. 16, 2008, page 892, XP002514950

## Description

The invention relates to a method of ¹³C-MR detection using an imaging medium comprising hyperpolarised ¹³C-acetate.

Magnetic resonance (NMR) imaging (MRI) is a technique that has become particularly attractive to physicians as images of a patients body or parts thereof can be obtained in a non-invasive way and without exposing the patient and the medical personnel to potentially harmful radiation such as X-rays. Because of its high quality images and good spatial and temporal resolution, MRI is a favourable imaging technique for imaging soft tissue and organs.

MRI may be carried out with or without MR contrast agents. However, contrast-enhanced MRI usually enables the detection of much smaller tissue changes which makes it a powerful tool for the detection of early stage tissue changes like for instance small tumours or metastases.

Several types of contrast agents have been used in MRI. Water-soluble paramagnetic metal chelates, for instance gadolinium chelates like Omniscan^{™} (GE Healthcare) are widely used MR contrast agents. Because of their low molecular weight they rapidly distribute into the extracellular space (i.e. the blood and the interstitium) when administered into the vasculature. They are also cleared relatively rapidly from the body.

Blood pool MR contrast agents on the other hand, for instance superparamagnetic iron oxide particles, are retained within the vasculature for a prolonged time. They have proven to be extremely useful to enhance contrast in the liver but also to detect capillary permeability abnormalities, e.g. "leaky" capillary walls in tumours which are a result of tumour angiogenesis.

WO-A-99/35508 discloses a method of MR investigation of a patient using a hyperpolarised solution of a high T₁ agent as MRI contrast agent. The term "hyperpolarisation" means enhancing the nuclear polarisation of NMR active nuclei present in the high T₁ agent, i.e. nuclei with non-zero nuclear spin, preferably ¹³C- or ¹⁵N-nuclei. Upon enhancing the nuclear polarisation of NMR active nuclei, the population difference between excited and ground nuclear spin states of these nuclei is significantly increased and thereby the MR signal intensity is amplified by a factor of hundred and more. When using a hyperpolarised ¹³ C- and/or ¹⁵N-enriched high T₁ agent, there will be essentially no interference from background signals as the natural abundance of ¹³C and/or ¹⁵N is negligible and thus the image contrast will be advantageously high. The main difference between conventional MRI contrast agents and these hyperpolarised high T₁ agents is that in the former changes in contrast are caused by affecting the relaxation times of water protons in the body whereas the latter class of agents can be regarded as non-radioactive tracers, as the signal obtained arises solely from the agent.

A variety of possible high T₁ agents for use as MR imaging agents are disclosed in WO-A-99/35508, including non-endogenous and endogenous compounds. As examples of the latter intermediates in normal metabolic cycles are mentioned which are said to be preferred for imaging metabolic activity. By *in vivo* imaging of metabolic activity, information of the metabolic status of a tissue may be obtained and said information may for instance be used to discriminate between healthy and diseased tissue.

Pyruvate for instance is a compound that plays a role in the citric acid cycle and the conversion of hyperpolarised ¹³C-pyruvate to its metabolites hyperpolarised ¹³C-lactate, hyperpolarised ¹³C-bicarbonate and hyperpolarised ¹³C-alanine can be used for *in vivo* MR studying of metabolic processes in the human body. Hyperpolarised ¹³C-pyruvate may for instance be used as an MR imaging agent for *in vivo* tumour imaging as described in detail in WO-A-2006/011810 and for assessing the viability of myocardial tissue by MR imaging as described in detail in WO-A-2006/054903.

WO-A-92/01937 discloses the measurement of the contribution of exogenously administered ¹³C labelled substrates to acetyl-CoA in vivo or in vitro, e.g. for substrate utilisation studies. The substrate can be 2-¹³C acetate or 1,2-¹³C acetate. After administration of the labelled compounds, the enrichment pattern in one or more metabolic products is measured.

The metabolic conversion of hyperpolarised ¹³C-pyruvate to its metabolites hyperpolarised ¹³C-lactate, hyperpolarised ¹³C-bicarbonate and hyperpolarised ¹³C-alanine can be used for *in vivo* MR study of metabolic processes in the human body since said conversion has been found to be fast enough to allow signal detection from the parent compound, i.e. hyperpolarised ¹³C-pyruvate, and its metabolites.

The amount of alanine, bicarbonate and lactate is dependent on the metabolic status of the tissue under investigation. The MR signal intensity of hyperpolarised ¹³C-lactate, hyperpolarised ¹³C -bicarbonate and hyperpolarised ¹³C-alanine is related to the amount of these compounds and the degree of polarisation left at the time of detection, hence by monitoring the conversion of hyperpolarised ¹³C-pyruvate to hyperpolarised ¹³C-lactate, hyperpolarised ¹³C-bicarbonate and hyperpolarised ¹³C-alanine it is possible to study metabolic processes *in vivo* in the human or non-human animal body by using non-invasive MR imaging or MR spectroscopy.

The MR signal amplitudes arising from the different pyruvate metabolites vary depending on the tissue type. The unique metabolic peak pattern formed by alanine, lactate, bicarbonate and pyruvate can be used as fingerprint for the metabolic state of the tissue under examination.

However, the production of hyperpolarised ¹³C-pyruvate which is suitable as an *in vivo* imaging agent is not without challenges. Hyperpolarised ⁱ³C-pyruvate is preferably obtained by dynamic nuclear polarisation (DNP) of either ¹³C-pyruvic acid or a ¹³C-pyruvate salt as described in detail in WO-A1-2006/011809.

The use of ¹³C-pyruvic acid simplifies the polarisation process since it does not crystallize upon freezing/cooling (crystallization leads to low dynamic nuclear polarisation or no polarisation at all). As a consequence no solvents and/or glass formers are needed to prepare a composition for the DNP process and thus a highly concentrated ¹³C-pyruvic acid sample can be used. However, due to its low pH a DNP agent needs to be used which is stable in the strong acid. Further, a strong base is necessary to dissolve and convert the solid hyperpolarised ¹³C-pyruvic acid after the polarisation to hyperpolarised ¹³C-pyruvate. Both the strong pyruvic acid and the strong base require careful selection of materials (e.g. dissolution medium reservoir, tubes, etc.) the compounds get in touch with.

Alternatively, a ¹³C-pyruvate salt may be used in the DNP process. Unfortunately, sodium ¹³C-pyruvate crystallizes upon freezing/cooling which makes it necessary to add glass formers. If the hyperpolarised ¹³C-pyruvate is intended to be used as *in vivo* imaging agent, the pyruvate concentration in the composition containing the pyruvate and glass formers is unfavourably low. Besides, the glass formers are to be removed for *in vivo* use as well.

Thus preferred salts which may be used for DNP are those ¹³C-pyruvates which comprise an inorganic cation from the group consisting of NH₄⁺, K⁺, Rb⁺, Cs⁺, Ca²⁺, Sr²⁺ and Ba²⁺, preferably NH₄⁺, K⁺, Rb⁺ or Cs⁺, more preferably K⁺, Rb⁺, Cs⁺ and most preferably Cs⁺, as in detail described in WO-A-2007/111515. Most of these salts are not commercially available and need to be synthesized separately. Further, if the hyperpolarised ¹³C-pyruvate is used *in vivo* MR imaging it is preferred to exchange the inorganic cation from the group consisting of NH₄⁺, K⁺, Rb⁺, Cs⁺, Ca²⁺ Sr²⁺ and Ba²⁺ by a physiologically very well tolerable cation like Na⁺ or meglumine. Hence an additional step is required after dissolution of the solid hyperpolarised ¹³C-pyruvate during which polarisation decays.

Other preferred salts are ¹³C-pyruvate of an organic amine or amino compound, preferably TRIS-¹³C₁-pyruvate or meglumine-¹³C₁-pyruvate, as in detail described in WO-A-2007/069909. Again these salts need to be synthesized separately.

Hence there is a need of alternative hyperpolarised imaging agents which can be used to obtain information about metabolic activity.

We have now found that hyperpolarised ¹³C-acetate may be used as such an imaging agent.

Sodium ¹³C-acetate is a commercially available compound which may be directly used for DNP since it does not crystallize upon cooling/freezing. Since this eliminates the necessity of glass formers and/or high amounts of solvent(s) in the sample, a highly concentrated sample can be prepared and used in the DNP process. Also, sodium ¹³C-acetate samples are just slightly basic and hence a variety of DNP agents can be used. Acetate is an endogenous compound which is very well tolerated and using hyperpolarised ¹³C-acetate as an imaging agent is advantageous from a safety perspective.

Compared to pyruvate, acetate may be used to get insight in different metabolic pathways than the former. Thus, acetate may be used to obtain information about the energy metabolism of fatty acids and for studying glycolysis. This information may be used to identify other disease states than those which can be identified by using pyruvate. Alternatively, this information may be combined with the information which is obtained by using pyruvate and thus help to identify diseases early and/or more precisely.

Thus, in a first aspect the invention provides a method of ¹³C-MR detection using an imagine medium comprising hyperpolarised ¹³C-acetate wherein signals of ¹³C-acetylcarnitine and optionally ¹³C-acetyl-CoA or ¹³C-acetyl-CoA and ¹³C-acetate are detected.

The term "signals of ¹³C-acetylcarnitine and optionally ¹³C-acetyl-CoA or ¹³C-acetyl-CoA and ¹³C-acetate are detected" means that in the method of the invention, only the signal of ¹³C-acetylcarnitine is detected or the signals of ¹³C-acetylcarnitine and ¹³C-acetyl-CoA are detected or the signals of ¹³C-acetylcarnitine and ¹³C-acetyl-CoA and ¹³C-acetate are detected.

The term "¹³C-MR detection" denotes ¹³C-MR imaging or ¹³C-MR spectroscopy or combined ¹³C-MR imaging and ¹³C-MR spectroscopy, i.e. ¹³C-MR spectroscopic imaging. The term further denotes ¹³C-MR spectroscopic imaging at various time points.

The term "imaging medium" denotes a liquid composition comprising hyperpolarised ¹³C-acetate as the MR active agent, i.e. imaging agent.

The imaging medium used in the method of the invention may be used as an imaging medium tor in vivo ¹³C-MR detection, i.e. in living human or non-human animal beings. Further, the imaging medium used in the method of the invention may be used as imaging medium for *in vitro* ¹³C-MR detection, e.g. of cell cultures, samples like for instance urine, saliva or blood, *ex vivo* tissue, for instance ex vivo tissue obtained from a biopsy or isolated organs, all of those derived from a living human or non-human animal body.

The term "¹³C-acetate" denotes a salt of ¹³C-acetic acid that is isotopically enriched with ¹³C, i.e. in which the amount of ¹³C isotope is greater than its natural abundance. Unless otherwise specified, the terms "¹³C-acetate" and "¹³C-acetic acid" denote a compound which is ¹³C-enriched at any of the two carbon atoms present in the molecule, i.e. at the C1-position and/or the C2-position.

The isotopic enrichment of the hyperpolarised ¹³C-acetate used in the method of the invention is preferably at least 75%, more preferably at least 80% and especially preferably at least 90%, an isotopic enrichment of over 90% being most preferred. Ideally, the enrichment is 100%. ¹³C-acetate used in the method of the invention may be isotopically enriched at the C1-position (in the following denoted ¹³C₁-acetate), at the C2-position (in the following denoted ¹³C₂-acetate) or at the C1- and the C2-position (in the following denoted ¹³C_{1,2}-acetate). Isotopic enrichment at the C1-position or at the C1- and the C2-position is preferred and isotopic enrichment at the C1-position is most preferred.

Further, deuterated ¹³C-acetate may be used in the method of the invention, i.e. one or more hydrogen atoms in ¹³C-acetate may be exchanged by deuterium atoms. In a preferred embodiment, ¹³C₁-acetate-d₃ is used in the method of the invention, i.e. ¹³C₁-acetate wherein all three hydrogen atoms of the methyl group are exchanged by deuterium atoms.

In a preferred embodiment, the imaging medium according to the invention comprises hyperpolarised ¹³C₁-acetate or ¹³C₁-acetate-d₃.

The term "¹³C-acetylcarnitine" denotes 3-acetyloxy-4-trimethylammoniobutanoate that is isotopically enriched with ¹³C, i.e. in which the amount of ¹³C isotope is greater than its natural abundance. Unless otherwise specified, the term "¹³C-acetylcarnitine" denotes a compound which is ¹³C-enriched at any of the two carbon atoms of the acetyloxy group, i.e. at the methyl group or the deuteromethyl group, if deuterated ¹³C₁-acetate was used in the method of the invention, and/or the carbonyl group of said acetyloxy group.

The term "¹³C-acetyl-CoA" denotes the thioester of coenzyme A and acetic acid which is isotopically enriched with ¹³C, i.e. in which the amount of ¹³C isotope is greater than its natural abundance. Unless otherwise specified, the term "¹³C-acetyl-CoA" denotes a compound which is ¹³C-enriched at any of the two carbon atoms of the acetyl group, i.e. at the methyl group, or the deuteromethyl group, if deuterated ¹³C₁-acetate was used in the method of the invention, and/or the carbonyl group of said acetyl group.

The terms "hyperpolarised" and "polarised" are used interchangeably hereinafter and denote a nuclear polarisation level in excess of 0.1%, more preferred in excess of 1 % and most preferred in excess of 10%.

The level of polarisation may for instance be determined by solid state ¹³C-NMR measurements in solid hyperpolarised ¹³C-acetate, e.g. solid hyperpolarised ¹³C-acetate obtained by dynamic nuclear polarisation (DNP) of ¹³C-acetate. The solid state ¹³C-NMR measurement preferably consists of a simple pulse-acquire NMR sequence using a low flip angle. The signal intensity of the hyperpolarised ¹³C-acetate in the NMR spectrum is compared with signal intensity of ¹³C-acetate in a NMR spectrum acquired before the polarisation process. The level of polarisation is then calculated from the ratio of the signal intensities of before and after polarisation.

In a similar way, the level of polarisation for dissolved hyperpolarised ¹³C-acetate may be determined by liquid state NMR measurements. Again the signal intensity of the dissolved hyperpolarised ¹³C-acetate is compared with the signal intensity of the dissolved ¹³C-acetate before polarisation. The level of polarisation is then calculated from the ratio of the signal intensities of ¹³C-acetate before and after polarisation. Hyperpolarisation of NMR active ¹³C-nuclei may be achieved by different methods which are for instance described in described in WO-A-98/30918, WO-A-99/24080 and WO-A-99/35508. Hyperpolarisation methods known in the art are polarisation transfer from a noble gas, "brute force", spin refrigeration, the parahydrogen method and dynamic nuclear polarisation (DNP).

To obtain hyperpolarised "¹³C-acetate, it is preferred to polarise ¹³C-acetate directly. Also ¹³C-acetic acid may be polarised, however the polarised ¹³C-acetic acid must subsequently be converted to polarised ¹³C-acetate, e.g. by neutralisation with a base, which is an additional step and hence this embodiment is less preferred. ¹³C-acetate salts, e.g. sodium ¹³C-acetate, are commercially available. ¹³C-acetic acid is commercially available as well; it can also be obtained by protonating a commercially available ¹³C-acetate, e.g. sodium ¹³C-acetate.

One way for obtaining hyperpolarised ¹³C-acetate is the polarisation transfer from a hyperpolarised noble gas which is described in WO-A-98/30918. Noble gases having non-zero nuclear spin can be hyperpolarised by the use of circularly polarised light. A hyperpolarised noble gas, preferably He or Xe, or a mixture of such gases, may be used to effect hyperpolarisation of ¹³C-nuclei. The hyperpolarised gas may be in the gas phase, it may be dissolved in a liquid/solvent, or the hyperpolarised gas itself may serve as a solvent. Alternatively, the gas may be condensed onto a cooled solid surface and used in this form, or allowed to sublime. Intimate mixing of the hyperpolarised gas with ¹³C-acetate or ¹³C-acetic acid is preferred.

Another way for obtaining hyperpolarised ¹³C-acetate is that polarisation is imparted to ¹³C-nuclei by thermodynamic equilibration at a very low temperature and high field. Hyperpolarisation compared to the operating field and temperature of the NMR spectrometer is effected by use of a very high field and very low temperature (brute force). The magnetic field strength used should be as high as possible, suitably higher than 1 T, preferably higher than 5 T, more preferably 15 T or more and especially preferably 20 T or more. The temperature should be very low, e.g. 4.2 K or less, preferably 1.5 K or less, more preferably 1.0 K or less, especially preferably 100 inK or less.

Another way for obtaining hyperpolarised ¹³C-acetate is the spin refrigeration method. This method covers spin polarisation of a solid compound or system by spin refrigeration polarisation. The system is doped with or intimately mixed with suitable crystalline paramagnetic materials such as Ni²⁺, lanthanide or actinide ions with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The prerequisite for this method is that the paramagnetic species has a highly anisotropic g-factor. As a result of the sample rotation, the electron paramagnetic resonance will be brought in contact with the nuclear spins, leading to a decrease in the nuclear spin temperature. Sample rotation is carried out until the nuclear spin polarisation has reached a new equilibrium.

In a preferred embodiment, DNP (dynamic nuclear polarisation) is used to obtain hyperpolarised ¹³C-acetate. In DNP, polarisation of MR active nuclei in a compound to be polarised is affected by a polarisation agent or so-called DNP agent, a compound comprising unpaired electrons. During the DNP process, energy, normally in the form of microwave radiation, is provided, which will initially excite the DNP agent. Upon decay to the ground state, there is a transfer of polarisation from the unpaired electron of the DNP agent to the NMR active nuclei of the compound to be polarised, e.g. to the ¹³C nuclei in ¹³C-acetate. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. The DNP technique is for example further described in WO-A-98/58272 and in WO-A-01196895.

To polarise a chemical entity, i.e. compound, by the DNP method, a composition of the compound to be polarised and a DNP agent is prepared which is then optionally frozen and inserted into a DNP polariser (where it will freeze if it has not been frozen before) for polarisation. After the polarisation, the frozen solid hyperpolarised composition is rapidly transferred into the liquid state either by melting it or by dissolving it in a suitable dissolution medium. Dissolution is preferred and the dissolution process of a frozen hyperpolarised composition and suitable devices therefore are described in detail in WO-A-02/37132. The melting process and suitable devices for the melting are for instance described in WO-A-02/36005.

In order to obtain a high polarisation level in the compound to be polarised said compound and the DNP agent need to be in intimate contact during the DNP process. This is not the case if the composition crystallizes upon being frozen or cooled. To avoid crystallization, either glass formers need to be present in the composition or compounds need to be chosen for polarisation which do not crystallize upon being frozen but rather form a glass.

In one embodiment, ¹³C-acetic acid, preferably ¹³C₁-acetic or ¹³C₁-acetic acid-d₃ (acetic acid-1-¹³C,2,2,2-d₃) is used as a starting material to obtain hyperpolarised ¹³C-acetate by the DNP method.

In a preferred embodiment, ¹³C-acetate, preferably ¹³C₁-acetate or ¹³C₁-acetate-d₃ is used as a starting material to obtain hyperpolarised ¹³C-acetate by the DNP method. Suitable ¹³C-acetates are sodium ¹³C-acetate and ¹³C-acetates which comprise an inorganic cation from the group consisting of NH₄⁺ K⁺, Rb⁺, Cs⁺, Ca²⁺, Sr²⁺ and Ba²⁺. The latter salts are described in detail in WO-A-2007/1111515. Alternatively, ¹³C-acetates of an organic amine or amino compound, preferably TRIS- ¹³C-acetate or meglumine-¹³C-acetate, may be used. These salts are in detail described in WO-A-2007/069909. In a most preferred embodiment, optionally deuterated TRIS-¹³C-acetate or optionally deuterated sodium ¹³C-acetate and even more preferably TRIS-¹³C₁-acetate, TRIS-¹³C,-acetate-d₃, sodium ¹³C₁-acetate or sodium ¹³C₁-acetate-d₃ is used as a starting material to obtain hyperpolarised ¹³C-acetate by the DNP method.

The term "TRIS" denotes 2-amino-2-hydroxymethyl-1,3-propanediol and the term ``TRIS-¹³C-acetate" denotes a salt which contains ¹³C-acetate as anion and a TRIS cation, i.e. TRIS ammonium (2-hydroxymethyl-1,3-propanedioyl ammonium).

For the hyperpolarisation of ¹³C-acetate by the DNP method, a composition is prepared which comprises ¹³C-acetate or ¹³C-acetic acid and a DNP agent.

The DNP agent plays a decisive role in the DNP process as its choice has a major impact on the level of polarisation that can be achieved in ¹³C-acetate. A variety of DNP agents - in WO-A-99/35508 denoted "OMRI contrast agents" - is known. The use of oxygen-based, sulphur-based or carbon-based stable trityl radicals as described in WO-A-99/35508, WO-A-88/10419, WO-A-90/00904, WO-A-91/12024, WO-A-93/02711 or WO-A-96/39367 has resulted in high levels of polarisation in a variety of different samples.

In a preferred embodiment, the hyperpolarised ¹³C-acetate used in the method of the invention is obtained by DNP and the DNP agent used is a trityl radical. As briefly mentioned above, the large electron spin polarisation of the DNP agent, i.e. trityl radical is converted to nuclear spin polarisation of ¹³C nuclei in ¹³C-acetate or ¹³C-acetic acid via microwave irradiation close to the electron Larmor frequency. The microwaves stimulate communication between electron and nuclear spin systems via e-e and e-n transitions. For effective DNP, i.e. to achieve a high level of polarisation in ¹³C-acetate or ¹³C-acetic acid, the trityl radical has to be stable and soluble in these compounds to achieve said intimate contact between ¹³C-acetate or ¹³C-acetic acid and the trityl radical which is necessary for the aforementioned communication between electron and nuclear spin systems.

In a preferred embodiment, the trityl radical is a radical of the formula (1) 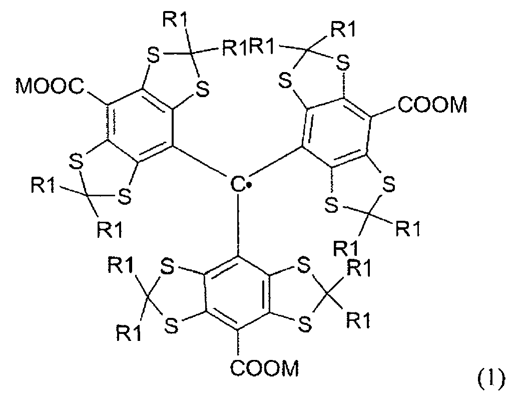 wherein
M represents hydrogen or one equivalent of a cation; and
R1 which is the same or different represents a straight chain or branched C₁-C₆-alkyl group optionally substituted by one or more hydroxyl groups or a group -(CH₂)ₙ-X-R2,
wherein n is 1, 2 or 3;
X is O or S; and
R2 is a straight chain or branched C₁-C₄-alkyl group, optionally substituted by one or more hydroxyl groups.

In a preferred embodiment, M represents hydrogen or one equivalent of a physiologically tolerable cation. The term "physiologically tolerable cation" denotes a cation that is tolerated by the human or non-human animal living body. Preferably, M represents hydrogen or an alkali cation, an ammonium ion or an organic amine ion, for instance meglumine. Most preferably, M represents hydrogen or sodium.

If ¹³C-acetate is used as a starting material to obtain Hyperpolarised ¹³C-acetate by the DNP method, R1 is preferably the same, more preferably a straight chain or branched C₁-C₄-alkyl group, most preferably methyl, ethyl or isopropyl; or R1 is preferably the same, more preferably a straight chain or branched C₁-C₄-alkyl group which is substituted by one hydroxyl group, most preferably -CH₂-CH₂-OH; or R1 is preferably the same and represents -CH₂-OC₂H₄OH.

If ¹³C-acetic acid is used as a starting material to obtain hyperpolarised ¹³C-acetate by the DNP method, R1 is the same or different, preferably the same and preferably represents -CH₂-OCH₃, -CH₂-OC₂H₅, -CH₂-CH₂-OCH₃, -CH₂-SCH₃, -CH₂-SC₂H₅ or -CH₂-CH₂-SCH₃, most preferably -CH₂-CH₂-OCH₃.

The aforementioned trityl radical of formula (1) may be synthesized as described in detail in WO-A-88/10419, WO-A-90/00904, WO-A-91/12024, WO-A-93/02711, WO-A-96/39367, WO-A-97/09633, WO-A-98/39277 and WO-A-2006/011811.

For the DNP process, a solution of the starting material, i.e. ¹³C-acetic acid or ¹³C-acetate (in the following denoted "sample") and the DNP agent, preferably a trityl radical, more preferably a trityl radical of formula (1) is prepared. A solvent or a solvent mixture may be used to promote dissolution of the DNP agent in the sample. However, if the hyperpolarised ¹³C-acetate is intended to be used as an imaging agent for *in vivo* ¹³C-MR detection, it is preferred to keep the amount of solvent to a minimum or, if possible, to avoid the use of solvents. To be used as an *in vivo* imaging agent, the polarised ¹³C-acetate is usually administered in relatively high concentrations, i.e. a highly concentrated sample is preferably used in the DNP process and hence the amount of solvent is preferably kept to a minimum. In this context, it is also important to mention that the mass of the composition containing the sample, i.e. DNP agent, sample and if necessary solvent, is kept as small as possible. A high mass will have a negative impact on the efficiency of the dissolution process, if dissolution is used to convert the solid composition containing the hyperpolarised ¹³C-acetic acid or ¹³C-acetate after the DNP process into the liquid state, e.g. for using it as an imaging agent for ¹³C-MR detection. This is due to the fact that for a given volume of dissolution medium in the dissolution process, the mass of the composition to dissolution medium ratio decreases, when the mass of the composition increases. Further, using certain solvents may require their removal before the hyperpolarised ¹³C-acetate used as an MR imaging agent is administered to a human or non-human animal being since they might not be physiologically tolerable.

If ¹³C-acetic acid is used as a starting material to obtain hyperpolarised ¹³C-acetate via DNP, a solution of the DNP agent, preferably a trityl radical and more preferably a trityl radical of formula (1) in ¹³C-acetic acid is prepared. ¹³C-acetic acid is a liquid at room temperature and the DNP agent is preferably dissolved in said liquid. Since ¹³C-acetic acid crystallizes upon freezing, a small amount of glass former, preferably glycerol, is added. Intimate mixing of the compounds can be promoted by several means known in the art, such as stirring, vortexing (whirl-mixing) or sonication and/or gentle heating.

If a ¹³C-acetate which is a solid at room temperature is used as a starting material to obtain hyperpolarised ¹³C-acetate via DNP, a solvent has to be added to prepare a solution of the DNP agent and the ¹³C-acetate. Preferably an aqueous carrier and most preferably water is used as a solvent. In one embodiment, the DNP agent is dissolved and the ¹³C-acetate is subsequently dissolved in the dissolved DNP agent. This embodiment is preferred if ¹³C-acetates which comprise an inorganic cation from the group consisting of NH₄⁺, K⁺, Rb⁺, Cs⁺, Ca^{2+,} Sr²⁺ and Ba₂⁺ and ¹³C-acetates of an organic amine or amino compound are used. In another embodiment, ¹³C-acetate is dissolved in the solvent and subsequently the DNP agent (as a dry compound or in solution) is added to and dissolved in the dissolved ¹³C-acetate. This embodiment is preferred when sodium ¹³C-acteate is used. In a more preferred embodiment, sodium ¹³C-acteate is dissolved in water and the solution is gently heated. This will lead to a super-saturated solution of relatively high viscosity which does not crystallize upon cooling/freezing. If the ¹³C-acetates mentioned above, i.e. sodium ¹³C-acetate, ¹³C-acetates which comprise an inorganic cation from the group consisting of NH₄⁺, K⁺, Rb⁺, Cs⁺, Ca²⁺, Sr²⁺ and Ba²⁺ and ¹³C-acetates of an organic amine or amino compound are used, no glass formers have to be added, since a composition containing these ¹³C-acetates does not crystallize upon cooling/freezing. Again intimate mixing of the compounds can be promoted by several means known in the art, such as stirring, vortexing or sonication and/or gentle heating.

If the hyperpolarised ¹³C-acetate used in the method of the invention is obtained by DNP, the composition to be polarised comprising ¹³C-acetic acid or ¹³C-acetate and a DNP agent may further comprise a paramagnetic metal ion. It has been found that the presence of paramagnetic metal ions may result in increased polarisation levels in the compound to be polarised by DNP as described in detail in WO-A2-2007/064226.

The term "paramagnetic metal ion" denotes paramagnetic metal ions in the form of their salts or in chelated form, i.e. paramagnetic chelates. The latter are chemical entities comprising a chelator and a paramagnetic metal ion, wherein said paramagnetic metal ion and said chelator form a complex, i.e. a paramagnetic chelate.

In a preferred embodiment, the paramagnetic metal ion is a salt or paramagnetic chelate comprising Gd³⁺, preferably a paramagnetic chelate comprising Gd³⁺. In a more preferred embodiment, said paramagnetic metal ion is soluble and stable in the composition to be polarised.

As with the DNP agent described before, the ¹³C-acetic acid or ¹³C-acetate to be polarised must be in intimate contact with the paramagnetic metal ion as well. The composition used for DNP comprising ¹³C-acetic acid or ¹³C-acetate, a DNP agent and a paramagnetic metal ion may be obtained in several ways. In a first embodiment the ¹³C-acetate is dissolved in a suitable solvent to obtain a solution; alternatively, liquid ¹³C-acetic acid as discussed earlier is used. To this solution of ¹³C-acetate or to the liquid ¹³C-acetic acid the DNP agent is added and dissolved. The DNP agent, preferably a trityl radical, might be added as a solid or in solution, preferably as a solid. In a subsequent step, the paramagnetic metal ion is added. The paramagnetic metal ion might be added as a solid or in solution, preferably as a solid. In another embodiment, the DNP agent and the paramagnetic metal ion are dissolved in suitable solvents or a suitable solvent and to this solution is added ¹³C-acetic acid or ¹³C-acetate. In yet another embodiment, the DNP agent (or the paramagnetic metal ion) is dissolved in a suitable solvent and added to ¹³C-acetic acid or ¹³C-acetate. In a subsequent step the paramagnetic metal ion (or the DNP agent) is added to this solution, either as a solid or in solution, preferably as a solid. Preferably, the amount of solvent to dissolve the paramagnetic metal ion (or the DNP agent) is kept to a minimum. Again intimate mixing of the compounds can be promoted by several means known in the art, such as stirring, vortexing or sonication and/or gentle heating.

If a trityl radical is used as DNP agent, a suitable concentration of such a trityl radical is 1 to 25 mM, preferably 2 to 20 mM, more preferably 10 to 15 mM in the composition used for DNP. If a paramagnetic metal ion is added to the composition, a suitable concentration of such a paramagnetic metal ion is 0.1 to 6 mM (metal ion) in the composition, and a concentration of 0.5 to 4 mM is preferred.

After having prepared a composition comprising ¹³C-acetic acid or ¹³C-acetate, the DNP agent and optionally a paramagnetic metal ion said composition is frozen by methods known in the art, e.g. by freezing it in a freezer, in liquid nitrogen or by simply placing it in the DNP polariser, where liquid helium will freeze it. In another embodiment, the composition is frozen as "beads" before it is inserted into to polariser. Such beads may be obtained by adding the composition drop wise to liquid nitrogen. A more efficient dissolution of such beads has been observed, which is especially relevant if larger amounts of ¹³C-acetic acid or ¹³C-acetate are polarised, for instance when it is intended to use the polarised ¹³C-acetate in an in vivo ¹³C-MR detection method.

If a paramagnetic metal ion is present in the composition said composition may be degassed before freezing, e.g. by bubbling helium gas through the composition (e.g. for a time period of 2 - 1 min) but degassing can be effected by other known common methods.

The DNP technique is for instance described in WO-A-98/58272 and in WO-A-01/96895. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. In a preferred embodiment, the DNP process is carried out in liquid helium and a magnetic field of about 1 T or above. Suitable polarisation units are for instance described in WO-A-02/37132. In a preferred embodiment, the polarisation unit comprises a cryostat and polarising means, e.g. a microwave chamber connected by a wave guide to a microwave source in a central bore surrounded by magnetic field producing means such as a superconducting magnet. The bore extends vertically down to at least the level of a region P near the superconducting magnet where the magnetic field strength is sufficiently high, e.g. between 1 and 25 T, for polarisation of the sample nuclei to take place. The bore for the probe (i.e. the frozen composition to be polarised) is preferably sealable and can be evacuated to low pressures, e.g. pressures in the order of 1 mbar or less. A probe introducing means such as a removable transporting tube can be contained inside the bore and this tube can be inserted from the top of the bore down to a position inside the microwave chamber in region P. Region P is cooled by liquid helium to a temperature low enough to for polarisation to take place, preferably temperatures of the order of 0.1 to 100 K, more preferably 0.5 to 10 K, most preferably to 5 K. The probe introducing means is preferably sealable at its upper end in any suitable way to retain the partial vacuum in the bore. A probe-retaining container, such as a probe-retaining cup, can be removably fitted inside the lower end of the probe introducing means. The probe-retaining container is preferably made of a light-weight material with a low specific heat capacity and good cryogenic properties such, e.g. KelF (polychlorotrifluoro-ethylene) or PEEK (polyetheretherketone) and it may be designed in such a way that it can hold more than one probe.

The probe is inserted into the probe-retaining container, submerged in the liquid helium and irradiated with microwaves. The microwave frequency may be determined from the EPR line of the DNP agent, which depends on the magnetic field of the magnet as 28.0 GHz/T. The optimal microwave frequency may be determined by adjusting the frequency for maximal NMR signal. Preferably, the optimal microwave frequency is in the about 94 GHz for a magnet charged to 3.35 T, 110 GHz for a magnet charged to 4 T, 140 GHz for a magnet charged to 5 T and 200 GHz for a magnet charged to 7 T. The power may be chosen between 50 and 200 mW, dependent on the probe size. The level of polarisation may be monitored as earlier described by for instance acquiring solid state ¹³C-NMR signals of the probe during microwave irradiation. Generally, a saturation curve is obtained in a graph showing NMR signal vs. time. Hence it is possible to determine when the optimal polarisation level is reached. A solid state ¹³C-NMR measurement suitably consists of a simple pulse-acquire NMR sequence using a low flip angle. The signal intensity of the dynamic nuclear polarised nuclei, i.e. ¹³C nuclei in ¹³C-acetic acid or ¹³C-acetate is compared with the signal intensity of the ¹³C nuclei in ¹³C-acetic acid or ¹³C-acetate before DNP. The polarisation is then calculated from the ratio of the signal intensities before and after DNP.

After the DNP process, the solid composition comprising the hyperpolarised ¹³C-acetic acid or ¹³C-acetate is transferred from a solid state to a liquid state, i.e. liquefied. This can be done by dissolution in an appropriate solvent or solvent mixture (dissolution medium) or by melting the solid composition. Dissolution is preferred and the dissolution process and suitable devices therefore are described in detail in WO-A-02/37132. The melting process and suitable devices for the melting are for instance described in WO-A-02/36005. Briefly, a dissolution unit/melting unit is used which is either physically separated from the polariser or is a part of an apparatus that contains the polariser and the dissolution unit/melting unit. In a preferred embodiment, dissolution/melting is carried out at an elevated magnetic field, e.g. inside the polariser, to improve the relaxation and retain a maximum of the hyperpolarisation. Field nodes should be avoided and low field may lead to enhanced relaxation despite the above measures.

If ¹³C-acetate has been used as the starting material for the dynamic nuclear polarisation and if the solid composition comprising the hyperpolarised ¹³C-acetate is liquefied by dissolution, an aqueous carrier, preferably a physiologically tolerable and pharmaceutically accepted aqueous carrier like water, a buffer solution or saline is suitably used as a solvent, especially preferably if the hyperpolarised ¹³C-acetate is intended for use in an imaging medium for *in vivo* ¹³C-MR detection. For *in vitro* applications also non aqueous solvents or solvent mixtures may be used, for instance DMSO or methanol or mixtures comprising an aqueous carrier and a non aqueous solvent, for instance mixtures of DMSO and water or methanol and water.

If ¹³C-acetic acid has been used as the starting material for the dynamic nuclear polarisation, the hyperpolarised ¹³C-acetic acid obtained has to be converted to ¹³C-acetate. If the solid composition comprising the hyperpolarised ¹³C-acetic acid is liquefied by dissolution, the dissolution medium is preferably an aqueous carrier, e.g. water or a buffer solution, preferably a physiologically tolerable buffer solution or it comprises an aqueous carrier, e.g. water or a buffer solution, preferably a physiologically tolerable buffer solution. The terms ``buffer solution" and "buffer" are hereinafter used interchangeably. In the context of this application ``buffer" denotes one or more buffers, i.e. also mixtures of buffers.

Preferred buffers are physiologically tolerable buffers, more preferably buffers which buffer in the range of about pH 7 to 8 like for instance phosphate buffer (KH₂PO₄/Na₂HPO₄), ACES, PIPES, imidazole/HCl, BES, MOPS, HEPES, TES, TRIS, HEPPS or TRICIN.

To convert hyperpolarised ¹³C-acetic acid into hyperpolarised ¹³C-acetate, ¹³C-acetic acid is generally reacted with a base. In one embodiment, ¹³C-acetic acid is reacted with a base to convert it to ¹³C-acetate and subsequently an aqueous carrier is added. In another preferred embodiment the aqueous carrier and the base are combined in one solution and this solution is added to ¹³C-acetic acid, dissolving it and converting it into ¹³C-acetate at the same time. In a preferred embodiment, the base is an aqueous solution of NaOH, Na₂CO₃ or NaHCO₃, most preferred the base is NaOH.

In another preferred embodiment, the aqueous carrier buffer or - where applicable - the combined aqueous carrier/base solution further comprises one or more compounds which are able to bind or complex free paramagnetic ions, e.g. chelating agents like DTPA or EDTA.

If hyperpolarisation is carried out by the DNP method, the DNP agent, preferably a trityl radical and the optional paramagnetic metal ion may be removed from the liquid containing the hyperpolarised ¹³C-acetate. Removal of these compounds is preferred if the hyperpolarised ¹³C-acetate is intended for use in an imaging medium for *in vivo* use. If ¹³C-acetic acid was as a starting material for DNP, it is preferred to first convert the hyperpolarised ¹³C-acetic acid into ¹³C-acetate and remove the DNP agent and the optional paramagnetic metal ion after said conversion has taken place.

Methods which are useful to remove the trityl radical and the paramagnetic metal ion are known in the art and described in detail in WO-A2-2007/064226 and WO-A1-2006/011809.

In a preferred embodiment the hyperpolarised ¹³C-acetate used in the method of the invention is obtained by dynamic nuclear polarisation of a composition that comprises TRIS-¹³C-acetate or sodium ¹³C-acetatc and more preferably TRIS-¹³C₁-acetate, TRIS-¹³C₁-acetate-d₃, sodium ¹³C₁-acetate or sodium ¹³C₁-acetate-d₃, a trityl radical of formula (1) and optionally a paramagnetic chelate comprising Gd³⁺. In this preferred embodiment, a solution of the trityl radical and, if used, the paramagnetic chelate comprising Gd³⁺ is prepared. The dissolved trityl radical and the optional dissolved paramagnetic chelate are added to sodium ¹³C-acetate or TRIS-¹³C-acetate and the composition is preferably sonicated or whirl-mixed and gently heated to promote intimate mixing of all the components.

The imaging medium according to the method of the invention may be used as imaging medium for *in vitro* ¹³C-MR detection, e.g. ¹³C-MR detection of cell cultures, samples, *ex vivo* tissue or isolated organs derived from the human or non-human animal body. For this purpose, the imaging medium is provided as a composition that is suitable for being added to, for instance, cell cultures, samples like urine, blood or saliva, *ex vivo* tissues like biopsy tissues or isolated organs. Such an imaging medium preferably comprises in addition to the imaging agent, i.e. hyperpolarised ¹³C-acetate, a solvent which is compatible with and used for *in vitro* cell or tissue assays, for instance DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

Further, the imaging medium according to the method of the invention may be used as imaging medium for *in vivo* ¹³C-MR detection, i.e. ¹³C-MR detection carried out on living human or non-human animal beings. For this purpose, the imaging medium needs to be suitable for administration to a living human or non-human animal body. Hence such an imaging medium preferably comprises in addition to the imaging agent, i.e. hyperpolarised ¹³C-acetate, an aqueous carrier, preferably a physiologically tolerable and pharmaceutically accepted aqueous carrier like water, a buffer solution or saline. Such an imaging medium may further comprise conventional pharmaceutical or veterinary carriers or excipients, e.g. formulation aids such as stabilizers, osmolality adjusting agents and solubilising agents which are conventional for diagnostic compositions in human or veterinary medicine.

If the imaging medium used in the method of the invention is used for *in vivo* ¹³C-MR detection, i.e. in a living human or non-human animal body, said imaging medium is preferably administered to said body parenterally, preferably intravenously. Generally, the body under examination is positioned in an MR magnet. Dedicated ¹³C-MR RF-coils are positioned to cover the area of interest. Exact dosage and concentration of the imaging medium will depend upon a range of factors such as toxicity and the administration route. Generally, the imaging medium is administered in a concentration of up to 1 mmol acetate per kg bodyweight, preferably 0.01 to 0.5 mmol/kg, more preferably 0.1 to 0.3 mmol/kg. At less than 400 s after the administration, preferably less than 120 s, more preferably less than 60 s after the administration, especially preferably 20 to 50 s an MR imaging sequence is applied that encodes the volume of interest in a combined frequency and spatial selective way. The exact time of applying an MR sequence is highly dependent on the volume of interest and of the species.

In the method according to the invention, signals of ¹³C-acetylcarnitine and optionally ¹³C-acetyl-CoA or ¹³C-acetyl-CoA and ¹³C-acetate are detected. In a preferred embodiment, signals of ¹³C-acetylcarnitine and ¹³C-acetyl-CoA are detected.

The metabolic conversion of acetate to acetyl-CoA and acetylcarnitine is shown in scheme 1 for ¹³C₁-acetate; * denotes the ¹³C-label : ¹³C-acetate reacts with coenzyme A to form ¹³C-acetyl-CoA in a reaction catalysed by acetyl CoA ligase (ACS, EC 6.2.1.1).

¹³C-acetyl-CoA then reacts with carnitine to ¹³C-acetylcarnitine and coenzyme A in a reaction catalysed by carnitine acetyltransferase (CAT, EC 2.3.1.7).

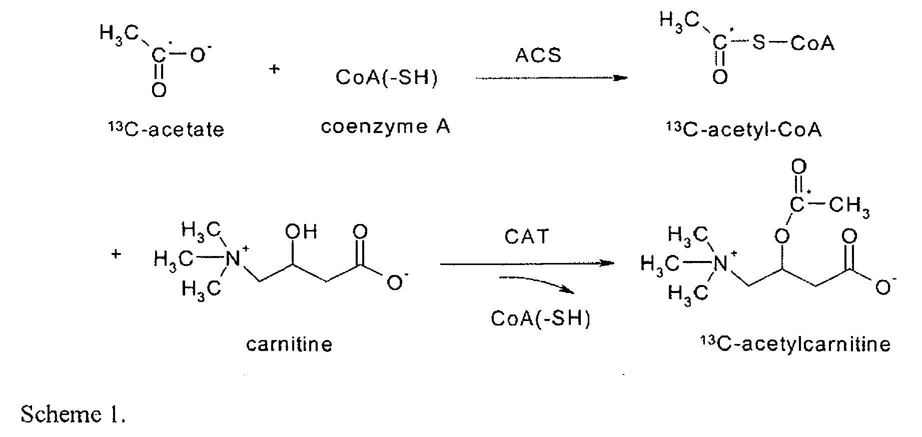

The term "signal" in the context of the invention refers to the MR signal amplitude or integral or peak area to noise of peaks in a ¹³C-MR spectrum which represent ¹³C-acetylcarnitine and optionally ¹³C-acetyl-CoA or ¹³C-acetyl-CoA and ¹³C-acetate. In a preferred embodiment, the signal is the peak area.

In a preferred embodiment of the method of the invention, the above-mentioned signals of ¹³C-acetylcarnitine and optionally ¹³C-acetyl-CoA or ¹³C-acetyl-CoA and ¹³C-acetate are used to generate a metabolic profile of a living human or non-human animal being. Said metabolic profile may be derived from the whole body, e.g. obtained by whole body *in vivo* ¹³C-MR detection. Alternatively, said metabolic profile is generated from a region or volume of interest, i.e. a certain tissue, organ or part of said human or non-human animal body.

In another preferred embodiment of the method of the invention, the above-mentioned signals of ¹³C-acetylcarnitine and optionally ¹³C-acetyl-CoA or ¹³C-acetyl-CoA and ¹³C-acetate are used to generate a metabolic profile of cells in a cell culture, of samples like urine, blood or saliva, of *ex vivo* tissue like biopsy tissue or of an isolated organ derived from a human or non-human animal being. Said metabolic profile is then generated by *in vitro* ¹³C-MR detection.

Thus in a preferred embodiment it is provided a method of ¹³C-MR detection using an imaging medium comprising hyperpolarised ¹³C-acetate and detecting signals of ¹³C-acetylcarnitine and optionally ¹³C-acetyl-CoA or ¹³C-acetyl-CoA and ¹³C-acetate, wherein said signals are used to generate a metabolic profile.

In a preferred embodiment, the signals of ¹³C-acetylcarnitine and ¹³C-acetyl-CoA are used to generate said metabolic profile.

In one embodiment, the spectral signal intensities of ¹³C-acetylcarnitine and optionally ¹³C-acetyl-CoA or ¹³C-acetyl-CoA and ¹³C-acetate are used to generate the metabolic profile. In another embodiment, the spectral signal integrals of ¹³C-acetylcarnitine and optionally ¹³C-acetyl-CoA or ¹³C-acetyl-CoA and ¹³C-acetate are used to generate the metabolic profile. In another embodiment, signal intensities from separate images of ¹³C-acetylcarnitine and optionally ¹³C-acetyl-CoA or ¹³C-acetyl-CoA and ¹³C-acetate are used to generate the metabolic profile. In yet another embodiment, the signal intensities of ¹³C-acetylcarnitine and optionally ¹³C-acetyl-CoA or ¹³C-acetyl-CoA and ¹³C-acetate are obtained at two or more time points to calculate the rate of change of ¹³C-acetylcarnitine and optionally ¹³C-acetyl-CoA or ¹³C-acetyl-CoA and ¹³C-acetate.

In another embodiment the metabolic profile includes or is generated using processed signal data of ¹³C-acetylcarnitine and optionally ¹³C-acetyl-CoA or ¹³C-acetyl-CoA and ¹³C-acetate, e.g. ratios of signals, corrected signals, or dynamic or metabolic rate constant information deduced from the signal pattern of multiple MR detections, i.e. spectra or images. Thus, in a preferred embodiment a corrected ¹³C-acetylcarnitine signal, i.e. ¹³C-acetylcarnitine to ¹³C-acetate signal or ¹³C-acetylcarnitine signal to ¹³C-acetyl-CoA signal is included into or used to generate the metabolic profile. In a further preferred embodiment, a corrected ¹³C-acetylcarnitine to total ¹³C-carbon signal is included into or used to generate the metabolic profile with total ¹³C-carbon signal being the sum of the signals of ¹³C-acetylcarnitine and optionally ¹³C-acetyl-CoA or ¹³C-acetyl-CoA and ¹³C-acetate. In a more preferred embodiment, the ratio of ¹³C-acetylcarnitine to ¹³C-acetyl-CoA is included into or used to generate the metabolic profile.

The metabolic profile generated in the preferred embodiment of the method according to the invention provides information about the metabolic activity of the body, part of the body, cells, tissue, body sample etc under examination and said information may be used in a subsequent step for, e.g. identifying diseases.

Such a disease may be a tumour since tumour tissue is usually characterized by a higher metabolic activity than healthy tissue. Such a higher metabolic activity would be apparent from comparing the metabolic profile of a tumour or of an *ex vivo* sample of a tumour with the metabolic profile of healthy tissue (e.g. surrounding tissue or healthy ex vivo tissue) and may manifest itself in the metabolic profile by high signals of ¹³C-acetylcarnitine and optionally ¹³C-acetyl-CoA or high corrected ¹³C-acetylcarnitine signal or high ratio of ¹³C-acetylcarnitine to ¹³C-acetyl-CoA or to ¹³C-acetate or total carbon or a high metabolic rate of ¹³C-acetylcarnitine build-up.

Another disease may be ischemia in the heart since ischemic myocardial tissue is usually characterized by a lower metabolic activity than healthy myocardial tissue. Again such a lower metabolic activity would be apparent from comparing the metabolic profile of ischemic myocardial tissue with the metabolic profile of healthy myocardial tissue in a similar way as described in the previous paragraph.

Another disease may be Alzheimer's disease or other diseases and disorders of the brain and/or related to brain function, cognition and/or memory since ¹³C-acetylcarnitine plays an enhanced central metabolic role in these diseases or disorders. It can be expected that a metabolic profile of a brain or parts thereof which is affected by these diseases or disorders which is obtained by the method of the invention is different from a metabolic profile from a healthy brain or parts thereof and that these diseases or disorders manifest themselves in the metabolic profile by high signals of ¹³C-acetylcarnitine and optionally ¹³C-acetyl-CoA or high corrected ¹³C-acetylcarnitine signal or high ratio of ¹³C-acetylcarnitine to ¹³C-acetyl-CoA or to ¹³C-acetate or total carbon or a high metabolic rate of ¹³C-acetylcarnitine build-up.

Another disease may be diabetes, since one of the central enzyme in the metabolism of acetate, carnitine acetyltransferase, plays a crucial role in fatty acid oxidation and is a promising target for the development of antidiabetes and antiobesity drugs. Suitably, the liver would be the organ of choice to generate a metabolic profile according to the method of the invention to identify diabetes and the disease manifests itself in said metabolic profile by high signals of ¹³C-acetylcarnitine and optionally ¹³C-acetyl-CoA or high corrected ¹³C-acetylcarnitine signal or high ratio of ¹³C-acetylcarnitine to ¹³C-acetyl-CoA or to ¹³C-acetate or total carbon or a high metabolic rate of ¹³C-acetylcarnitine build-up.

Yet another disease may be liver related diseases, such as liver fibrosis or liver cirrhosis. In these diseases an increased amount of esterified carnitine is characteristic and thus a metabolic profile of a diseased liver would show high signals of ¹³C-acetylcarnitine and optionally ¹³C-acetyl-COA or high corrected ¹³C-acetylcarnitine signal or high ratio of ¹³C-acetylcarnitine to ¹³C-acctyl-CoA or to ¹³C-acetate or total carbon or a high metabolic rate of ¹³C-acetylcarnitine build-up.

Anatomical and/or - where suitable - perfusion information may be included in the method of the invention for identification of diseases. Anatomical information may for instance be obtained by acquiring a proton or ¹³C-MR image with or without employing a suitable contrast agent before or after the method of the invention.

If the disease is ischemia in the heart, the relative perfusion in the myocardium can be determined by using an MR contrast agent like for instance Omniscan^{™}. Likewise, MR imaging techniques for perfusion measurement without the administration of a contrast agent are known in the art. In a preferred embodiment, a non-metabolised hyperpolarised ¹³C-contrast agent is used to determine quantitative perfusion. Suitable techniques and contrast agents are for instance described in WO-A-02/23209.

In another preferred embodiment, the imaging medium comprising Hyperpolarised ¹³C-acetate is administered repeatedly, thus allowing dynamic studies. This is a further advantage of the method according to the invention compared to other MR detection methods using conventional MR contrast agents which - in higher doses - may show toxic effects. Due to the low toxicity of acetate and its favourable safety profile, repeated doses of this compound are well tolerated by the patient.

As stated above, the metabolic profile provides information about the metabolic activity of the body, part of the body, cells, tissue, body sample etc. under examination and said information may be used in a subsequent step for, e.g. identifying diseases. However, a physician may also use this information in a further step to choose the appropriate treatment for the patient under examination.

Further, said information may be used to monitor treatment response, e.g. treatment success, of the above mentioned diseases, and its sensitivity makes the method especially suitable for monitoring early treatment response, i.e. response to treatment shortly after its commencement.

In another embodiment, the method of the invention may be used to assess drug efficacy. In said embodiment, potential drugs for curing a certain disease may be tested at a very early stage in drug screening, for instance *in vitro* in a cell culture which is a relevant model for said certain disease or in diseased *ex vivo* tissue or a diseased isolated organ. Alternatively, potential drugs for curing a certain disease may be tested at an early stage in drug screening *in vivo,* for instance in an animal model which is relevant for said certain disease. By comparing the metabolic profile of said cell culture, *ex vivo* tissue, isolated or test animal before and after treatment with a potential drug, the efficacy of said drug and thus treatment response and success can be determined which of course provides valuable information in the screening of potential drugs.

Also disclosed is a composition comprising sodium ¹³C₁-acetate, sodium ¹³C₁-acetate-d₃, TRIS-¹³C₁-acetate-d₃, ¹³C₁-acetic acid or ¹³C₁-acetic acid-d₃, a trityl radical and optionally a paramagnetic metal ion.

In a first disclosure, said composition comprises sodium ¹³C₁-acetate or sodium ¹³C₁-acetate-d₃ or TRIS-¹³C₁-acctate-d₃, a trityl radical and optionally a paramagnetic metal ion. In a preferred embodiment, said trityl radical is a trityl radical of formula (1) wherein M represents hydrogen or sodium and R1 is preferably the same, more preferably a straight chain or branched C₁-C₄-alkyl group, most preferably ethyl, ethyl or isopropyl; or R1 is preferably the same, more preferably a straight chain or branched C₁-C₄-alkyl group which is substituted by one hydroxyl group, most preferably -CH₂-CH₂-OH; or R1 is preferably the same and represents -CH₂-OC₂H₄OH. In another preferred embodiment said composition comprises a paramagnetic metal ion, preferably a salt or paramagnetic chelate comprising Gd³⁺ and more a paramagnetic chelate comprising Gd³⁺. Suitably, said composition further comprises a solvent or solvents; preferably an aqueous carrier and most preferably water is used as a solvent. The aforementioned compositions can be used for obtaining hyperpolarised sodium ¹³C₁-acetate or sodium ¹³C₁-acetate-d₃ or TRIS-¹³C₁-acetate-d₃ by dynamic nuclear polarisation (DNP) with a high polarisation level.

In a second disclosure, said composition comprises ¹³C₁-acetic acid or ¹³C₁-acetic acid-d₃, a trityl radical and optionally a paramagnetic metal ion. In a preferred embodiment, said trityl radical is a trityl radical of formula (1) wherein M represents hydrogen or sodium and R1 is the same or different, preferably the same and preferably represents -CH₂-OCH₃, -CH₂-OC₂H₅, -CH₂-CH₂-OCH₃, -CH₂-SCH₃, - CH₂-SC₂H₅ or -CH₂-CH₂-SCH₃, most preferably -CH₂-CH₂-OCH₃. In another preferred embodiment said composition comprises a paramagnetic metal ion, preferably a salt or paramagnetic chelate comprising Gd³⁺ and more a paramagnetic chelate comprising Gd³⁺. Said composition may or may not comprise a solvent or solvents; in a preferred embodiment an aqueous carrier and most preferably water is used as a solvent. The aforementioned compositions can be used for obtaining hyperpolarised ¹³C₁-acetic acid or hyperpolarised ¹³C₁-acetic acid-d₃ by dynamic nuclear polarisation (DNP) with a high polarisation level. Said hyperpolarised ¹³C₁-acetic acid or hyperpolarised ¹³C₁-acetic acid-d₃ can be converted into hyperpolarised ¹³C₁-acetate or hyperpolarised ¹³C₁-acetate-d₃ by dissolution with a base, e.g. NaOH.

Also disclosed is a composition comprising hyperpolarised sodium ¹³C₁-acetate, hyperpolarised sodium ¹³C₁-acetate-d₃, hyperpolarised TRIS-¹³C₁-acetate-d₃, hyperpolarised ¹³C₁-acetic acid or hyperpolarised ¹³C₁-acetic acid-d₃, a trityl radical and optionally a paramagnetic metal ion, wherein said composition is obtained by dynamic nuclear polarisation.

Also disclosed is hyperpolarised sodium ¹³C₁-acetate-d₃, hyperpolarised TRIS-¹³C₁-acetate-d₃, hyperpolarised ¹³C₁-acetic acid or hyperpolarised ¹³C₁-acetic acid-d₃. A preferred disclosure of this aspect is hyperpolarised sodium ¹³C₁-acetate-d₃ or hyperpolarised TRIS-¹³C₁-acetate-d₃, which can be used as imaging agent in a composition (imaging medium) for use in a ¹³C-MR detection method.

Also disclosed is an imaging medium comprising hyperpolarised sodium ¹³C₁-acetate-d₃ or hyperpolarised TRIS-¹³C₁-acetate-d₃.

The imaging medium for use in the method according to the invention may be used as imaging medium in a method of ¹³C-MR detection.

The imaging medium according to the method of the invention may be used as imaging medium for *in vitro* ¹³C-MR detection, e.g. ¹³C-MR detection of cell cultures, samples, *ex vivo* tissue or isolated organs derived from the human or non-human animal body. For this purpose, the imaging medium is provided as a composition that is suitable for being added to, for instance, cell cultures, samples like urine, blood or saliva, *ex vivo* tissues like biopsy tissues or isolated organs. Such an imaging medium preferably comprises in addition to the imaging agent, i.e. hyperpolarised sodium ¹³C₁-acetate-d₃ or hyperpolarised TRIS-¹³C₁-acetate-d₃, a solvent which is compatible with and used for *in vitro* cell or tissue assays, for instance DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

Further, the imaging medium according to the method of the invention may be used as imaging medium for *in vivo* ¹³C-MR detection, i.e. ¹³C-MR detection carried out on living human or non-human animal beings. For this purpose, the imaging medium needs to be suitable for administration to a living human or non-human animal body. Hence such an imaging medium preferably comprises in addition to the imaging agent, i.e. sodium ¹³C₁-acetate-d₃ or hyperpolarised TRIS-¹³C₁-acetate-d₃, an aqueous carrier, preferably a physiologically tolerable and pharmaceutically accepted aqueous carrier like water, a buffer solution or saline. Such an imaging medium may further comprise conventional pharmaceutical or veterinary carriers or excipients, e.g. formulation aids such as stabilizers, osmolality adjusting agents, and solubilising agents which are conventional for diagnostic compositions in human or veterinary medicine.

### Brief description of the drawings:

FIG. 1 depicts the build-up and decay of ¹³C1-acetate and ¹³C₁-acetylcarnitine signal over time. Data were collected with a surface coil placed over the mouse heart.
FIG. 2 depicts signal intensities of ¹³C₁-acetate, ¹³C₁-acetyl-CoA and ¹³C₁-acetylcarnitine taken 10 s after an intravenous injection of hyperpolarised ¹³C₁-acetate. Data were collected with a surface coil placed over the mouse heart.
FIG. 3 depicts the ratios of ¹³C₁-acetylcarnitine to ¹³C₁-acetyl-CoA measured in mouse heart and liver with a surface coil placed on these organs.
FIG. 4 depicts the ratios of ¹³C₁-acetylcarnitine to ¹³C₁-acetate measured in a hind leg of a mouse, before an ischemic period of 30 min, 5 min after the ischemic period and 1 hour after the ischemic period.

The invention is illustrated by the following examples.

### Examples

### Example 1a Production of hyperpolarised sodium ¹³C₁-acetate by the DNP method in the presence of a Gd-chelate as paramagnetic metal ion and a trityl radical as DNP agent

To a micro test tube was added sodium ¹³C₁-acetate (Aldrich, 24.9 mg, 0.30 mmol) and 16 µl water. The test tube was gently heated and sonicated to dissolve the sodium ¹³C₁-acetate. An aqueous solution of tris(8-carboxy-2,2,6,6-(tetra(hydroxyethyl)-benzo-[1,2-4,5']-bis-(1,3)-dithiole-4-yl)-methyl sodium salt (trityl radical) which had been synthesised according to Example 7 of WO-A1-98/39277 was prepared (139 µmol/g solution) and 3.4 mg of this solution were added to the dissolved sodium ¹³C₁-acetate in the tube. Further, an aqueous solution of the Gd-chelate of 1,3.5-tris-(N-(DO3A-acetamido)-N-methyl-4-amino-2-methylphenyl)-[1,3,5]tria-zinane-2,4,6-trione (paramagnetic metal ion) which had been synthesised according to Example 4 of WO-A-2007/064226 was prepared (14.6 µmol/g solution) and 1.2 mg of this solution was added to the test tube with the sodium ¹³C₁-acetate and the trityl radical. The resulting composition was sonicated and gently heated to dissolve all compounds. The composition (37 µl, 12.5 mM in trityl radical and 1.41 mM in Gd³⁺) was transferred from the tube to a sample cup and the sample cup was inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be more than 20 %.

### Example 1b Production of an imaging medium comprising hyperpolarised sodium ¹³C₁-acetate

After 75 minutes of dynamic nuclear polarisation, the obtained frozen polarised composition was dissolved in 6 ml phosphate buffer (20 mM, pH 7, 100 mg/l EDTA, 0.9% NaCl). The pH of the final solution containing the dissolved composition was 7.3 ± 0.1. The sodium ¹³C₁-acetate concentration in said final solution was 50 mM.

Liquid state polarisation was determined by liquid state ¹³C-NMR at 400 MHz to be 19 %.

### Example 2a Preparation of TRIS-¹³C₁-acetate

¹³C₁-acetic acid (Aldrich, 474 mg, 7.7 mmol) was dissolved in 50 ml water. To the solution was added TRIS (946 mg, 7.80 mmol). After the dissolution of the solid the solution was diluted in 200 ml water and freeze dried. The freeze-dried product TRIS-¹³C₁-acetate was characterized by NMR: purity 93%, 1.12 eq. TRIS.

### Example 2b Production of hyperpolarised TRIS-¹³C₁-acetate by the DNP method in the presence of a Gd-chelate as paramagnetic metal ion and a trityl radical as DNP agent

To a micro test tube were added TRIS-¹³C₁-acetate which was prepared according to Example 2a (54.6 mg, 0.30 mmol) and 10 µl water. The test tube was gently heated and sonicated to dissolve TRIS-¹³C₁-acetate. An aqueous solution of tris(8-carboxy-2,2,6,6-(tetra(hydroxyethyl)-benzo-[1,2-4,5']-bis-(1,3)-dithiole-4-yl)-methyl sodium salt (trityl radical) which had been synthesised according to Example 7 of WO-A1-98/39277 was prepared (139 µmol/g solution) and 6.0 mg of this solution were added to the dissolved TRIS-¹³C₁-acetate in the tube. Further, an aqueous solution of the Gd-chelate of 1,3,5-tris-(N-(D03A-acetamido)-N-methyl-4-amino-2-methylphenyl)-[1,3,5]tria-zinane-2,4,6-trione (paramagnetic metal ion) which had been synthesised according to Example 4 of WO-A-2007/064226 was prepared (14.6 µmol/g solution) and 1.3 mg of this solution was added to the test tube with the TRIS-¹³C₁-acetate and the trityl radical. The resulting composition was sonicated and gently heated to dissolve all compounds. The composition (65 µl, 12.8 mM in trityl radical and 0.9 mM in Gd³⁺) was transferred from the tube to a sample cup and the sample cup was inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be more than 25 %.

### Example 2c Production of an imaging medium comprising hyperpolarised TRIS-¹³C₁-acetate

After 75 minutes of dynamic nuclear polarisation, the obtained frozen polarised composition was dissolved in 6 ml phosphate buffer (20 mM, pH 7, 100 mg/l EDTA, 0.9% NaCl). The pH of the final solution containing the dissolved composition was 7.3 ±0.1. The TRIS-¹³C₁-acetate concentration in said final solution was 50 mM.

### Example 3a Production of hyperpolarised TRIS-¹³C-actetate-d₃ by the DNP method in the presence of a Gd-chelate as paramagnetic metal ion and a trityl radical as DNP agent

To a micro test tube were added TRIS-¹³C₁-actetate-d₃ which was prepared as described in Example 2a (55.5 mg, 0.30 mmol) and 10 µl water. The test tube was gently heated and sonicated to dissolve the TRIS-¹³C₁-actetate-d₃. An aqueous solution of tris(8-carboxy-2,2,6,6-(tetra(hydroxyethyl)-benzo-[1,2-4,5']-bis-(1,3)-dithiole-4-yl)-methyl sodium salt (trityl radical) which had been synthesised according to Example 7 of WO-A1-98/39277 was prepared (139 µmol/g solution) and 6.0 mg of this solution were added to the dissolved TRIS-¹³C₁-actetate-d₃ in the tube. Further, an aqueous solution of the Gd-chelate of 1,3,5-tris-(N-(DO3A-acetamido)-N-methyl-4-amino-2-methylphenyl)-[1,3,5]tria-zinane-2,4,6-trione (paramagnetic metal ion) which had been synthesised according to Example 4 of WO-A-2007/064226 was prepared (14.6 µmol/g solution) and 1.3 mg of this solution was added to the test tube with the TRIS-¹³C₁-actetate-d₃ and the trityl radical. The resulting composition was sonicated and gently heated to dissolve all compounds. The composition (65 µl, 12.8 mM in trityl radical and 0.9 mM in Gd³⁺) was transferred from the tube to a sample cup and the sample cup was inserted into a DNP polariser. The composition was polarised under DNP conditions at 1.2 K in a 3.35 T magnetic field under irradiation with microwave (93.89 GHz). Polarisation was followed by solid state ¹³C-NMR and the solid state polarisation was determined to be more than 25 %.

### Example 3b Production of an imaging medium comprising hyperpolarised TRIS-¹³C₁-actetate-d₃

After 75 minutes of dynamic nuclear polarisation, the obtained frozen polarised composition was dissolved in 6 ml phosphate buffer (20 mM, pH 7, 100 mg/l EDTA, 0.9% NaCl). The pH of the final solution containing the dissolved composition was 7.3 ± 0.1. The TRIS-¹³C₁-actetate-d₃ concentration in said final solution was 50 mM.

Liquid state polarisation was determine by liquid state ¹³C-NMR at 400 MHz to be 32 %.

### Example 4 In vivo ¹³C-MR spectroscopy in mice (heart) using an imaging medium comprising hyperpolarised TRIS-¹³C₁-acetate

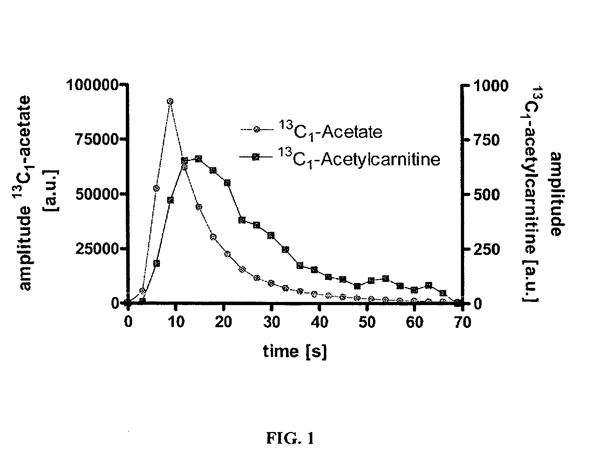

200 µl of an imaging medium which was prepared as described in Example 3a was injected into a C57B1/6 mouse over a time period of 6 s. The TRIS-¹³C₁-acetate concentration in said imaging medium was 75 mM and the polarisation was 20% at the time of injection. A ¹³C-surface coil (diameter 12 mm) was placed over the mouse myocardium. A rat ¹H-body coil was used for anatomical reference images and localised shimming. ¹³C-MR spectroscopy was carried out with a 2.4 T Bruker spectrometer. A dynamic set of ¹³C-MR spectra (in total 30) was acquired every 3 s with a 30 degree RF pulse. From this experiment a ¹³C₁-acetylcarnitine signal build-up over the time of the experiment was clearly seen (FIG. 1).

### Example 5 In vivo ¹³C-MR spectroscopy in mice (heart) using an imaging medium comprising hyperpolarised TRIS-¹³C₁-acetate

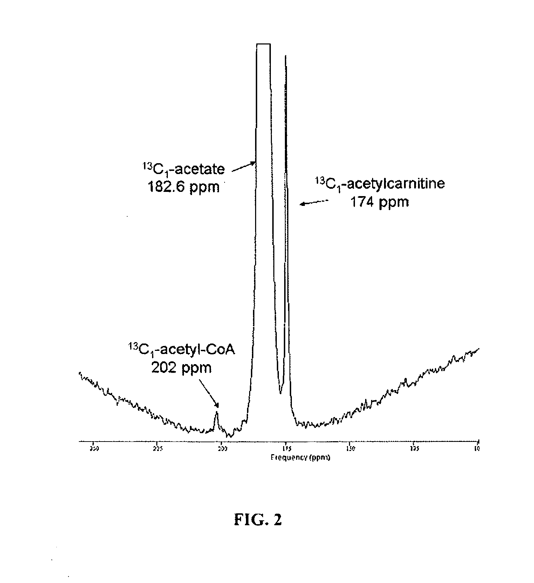

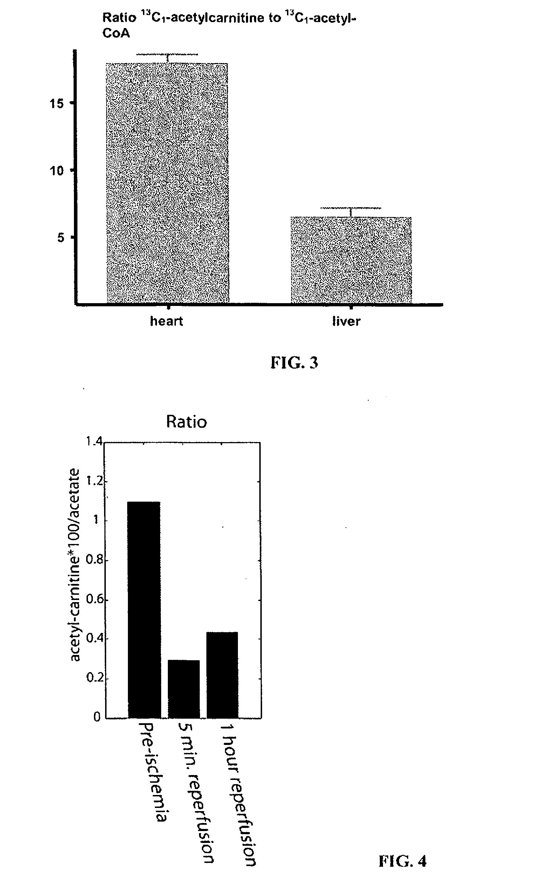

200 µl of an imaging medium which was prepared as described in Example 3a was injected into a C57B1/6 mouse over a time period of 6 s. The TRIS-¹³C₁-acetate concentration in said imaging medium was approx. 50 mM. In both experiments (n = 2) a ¹³C-surface coil (diameter 12 mm) was placed over the mouse myocardium and ¹³C-MR spectroscopy was carried out in a 2.4 T Bruker spectrometer. A spectrum was acquired 10 s after the imaging medium had been injected with a 90 degree RF pulse. From these two experiments both ¹³C₁-acetyl-CoA and ¹³C₁-acetylcarnitine could be identified (FIG 2) and quantified (FIG 3). The ratio of ¹³C₁-acetylcarnitine to ¹³C₁-acetyl-CoA was roughly 3 times higher in the heart than in the liver, see Example 6.

### Example 6 In vivo ¹³C-MR spectroscopy in mice (liver) using an imaging medium comprising hyperpolarised TRIS-¹³C₁-acetate

200 µl of an imaging medium which was prepared as described in Example 3a was injected into a C57B1/6 mouse over a time period of 6 s. The TRIS-¹³C₁-acetate concentration in said imaging medium was approx. 50 mM. In both experiments (n = 2) a ¹³C-surface coil (diameter 12 mm) was placed over the mouse liver and ¹³C-MR spectroscopy was carried out in a 2.4 T Bruker spectrometer. A spectrum was acquired 10 s after the imaging medium had been injected with a 90 degree RF pulse. From these two experiments both ¹³C₁-acetyl-CoA and ¹³C₁-acetylcarnitine could be identified (FIG 2) and quantified (FIG 3). The ratio of ¹³C₁-acetylcarnitine to ¹³C₁-acetyl-CoA was roughly 3 times lower in the heart than in the liver, see Example 5.

### Example 7 In vivo ¹³C-MR spectroscopy in mice (skeletal muscle) using an imaging medium comprising hyperpolarised TRIS-¹³C₁-acetate

200 µl of an imaging medium which was prepared as described in Example 3a was injected into a C57B1/6 mouse over a time period of 6 s. The TRIS-¹³C₁-acetate concentration in said imaging medium was approx. 50 mM. A ¹³C-surface coil (diameter 9 mm) was placed over the mouse hind leg and ¹³C-MR spectroscopy was carried out in a 2.4 T Bruker spectrometer. 20 pulses with 25° flip angle were acquired every 4 s. Three experiments were conducted: 1) before an ischemic period of 30 min, 2) 5 min after the ischemic period and 3) 1 hour after the ischemic period. From these experiments a ratio between ¹³C₁-acetylcarnitine and ¹³C₁-acetate could be quantified, see FIG. 4. A decrease of the ¹³C₁-acetylcarnitine signal of about a factor of 10 was observed right after the ischemic period and after one hour of reperfusion the signal was still very low compared to the control muscle.

## Claims

1. Method of ¹³C-MR detection using an imaging medium comprising hyperpolarised ¹³C-acetate wherein signals of ¹³C-acetylcarnitine and optionally ¹³C-acetyl-CoA or ¹³C-acetyl-CoA and ¹³C-acetate are detected.

2. The method according to claim 1 wherein signals of ¹³C-acetylcarnitine and ¹³C-acetyl-CoA are detected.

3. The method according to claims 1 or 2 wherein said signals are used to generate a metabolic profile.

4. The method according to claim 3, wherein said method is a method of *in vivo* ¹³C-MR detection and said metabolic profile is a metabolic profile of a living human or non-human animal being

5. The method according to claim 3, wherein said method is a method of *in vitro* ¹³C-MR detection and said metabolic profile is a metabolic profile of cells in a cell culture, of samples, of *ex vivo* tissue or of an isolated organ derived from a human or non-human animal being.

## Patentansprüche

1. Verfahren zur ¹³C-MR-Erfassung mittels eines Bildgebungsmediums, das hyperpolarisiertes ¹³C-Acetat umfasst, wobei Signale von ¹³C-Acetylcarnitin und gegebenenfalls ¹³C-Acetyl-CoA oder ¹³C-Acetyl-CoA und ¹³C-Acetat erfasst werden.

2. Verfahren nach Anspruch 1, wobei Signale von ¹³C-Acetylcarnitin und ¹³C-Acetyl-CoA erfasst werden.

3. Verfahren nach Anspruch 1 oder 2, wobei die Signale verwendet werden, um ein metabolisches Profil zu erzeugen.

4. Verfahren nach Anspruch 3, wobei das Verfahren ein Verfahren zur *in vivo* ¹³C-MR-Erfassung ist und das metabolische Profil ein metabolisches Profil von einem lebenden menschlichen oder nicht menschlichen Lebewesen ist.

5. Verfahren nach Anspruch 3, wobei das Verfahren ein Verfahren zur *in vitro* ¹³C-MR-Erfassung ist und das metabolische Profil ein metabolisches Profil von Zellen in einer Zellkultur, von Proben, von *ex vivo* Gewebe oder von einem isolierten Organ ist, stammend von einem menschlichen oder nicht menschlichen Lebewesen.

## Revendications

1. Procédé de détection par résonance magnétique (RM) de ¹³C en utilisant un substrat d'imagerie comprenant de l'acétate marqué par ¹³C dans lequel des signaux d'acétylcarnitine marqué par ¹³C et, en variante, d'acétyl-CoA marqué par ¹³C ou d'acétyl-CoA marqué par ¹³C et d'acétate marqué par 13C sont détectés.

2. Procédé selon la revendication 1, dans lequel des signaux d'acétylcarnitine marqué par ¹³C et d'acétyl-CoA marqué par ¹³C sont détectés.

3. Procédé selon les revendications 1 ou 2, dans lequel lesdits signaux sont utilisés de manière à produire un profil métabolique.

4. Procédé selon la revendication 3, dans lequel ledit procédé est un procédé in vivo de détection par RM de ¹³C et ledit profil métabolique est un profil métabolique d'un être animal, humain ou non humain, vivant.

5. Procédé selon la revendication 3, dans lequel ledit procédé est un procédé de détection par RM de ¹³C in vitro et ledit profil métabolique est un profil métabolique de cellules dans une culture cellulaire, d'échantillons, de tissu ex vivo ou d'un organe isolé issu d'un être animal, humain ou non humain.
